# EUROPEAN PATENT APPLICATION

(11) **EP 3 919 437 A1**
(43) Date of publication of application: **08.12.2021**
(21) Application number: 19912645.9
(22) Date of filing: 31.01.2019
(51) Int. Cl.: C01B 3/02

(54) **HYDROGEN-MIXED GAS GENERATION DEVICE AND HYDROGEN-MIXED GAS GENERATION METHOD**

(71) Applicant: Friend Co., Ltd., Kitakyushu-shi, Fukuoka 802-0002 (JP)
(72) Inventor: MURATA, Hideki, Kitakyushu-shi, Fukuoka 807-1263 (JP); HIDAKA, Naoki, Kitakyushu-shi, Fukuoka 807-1263 (JP)
(74) Representative: Hoffmann Eitle
(86) International application number: PCT/JP2019/003484
(87) International publication number: WO 2020/157936

(57) **Abstract**

A hydrogen mixed gas generation apparatus 10 comprises a warm water containment part 110 that contains warm water 200 that is set at a predetermined temperature, a gas supply conduit 120 that guides a hydrogen gas that is generated by a predetermined means into the warm water in the warm water containment part 110, and a gas discharge conduit 130 that guides and releases the hydrogen gas that is discharged into the warm water 200 by the gas supply conduit 120 from the warm water containment part 110 to an outside thereof. Furthermore, the hydrogen mixed gas generation apparatus 10 further comprises a hydrogen generation part 12 that executes one process of an electrolysis process for water, an overheat process for water vapor, or a chemical process to generate a hydrogen gas. Furthermore, the warm water is maintained at 50 to 90°C.

## Description

### Field

A disclosed embodiment(s) relate(s) to a hydrogen mixed gas generation apparatus and a generation method for a hydrogen mixed gas.

### Background

It is conventionally known that it is effective to incorporate hydrogen into a human body in order to remove an active oxygen species that is considered as a cause that causes a lesion and/or dysfunction. Hence, a hydrogen mixed gas generation apparatus is proposed that is capable of generating a hydrogen gas from a saturated vapor and incorporating such a hydrogen gas into a human body (see, for example, Patent Literature 1).

### Citation List

### Patent Literature

Patent Literature 1: Japanese Patent Application Publication No. 2013-151400

### Summary

### Technical Problem

However, support for an idea that a hydrogen gas that is obtained by a conventional hydrogen mixed gas generation apparatus is capable of attaining health promotion effectively has not been acquired.

An aspect of an embodiment is provided by taking the above into consideration and aims to provide a hydrogen mixed gas generation apparatus and a generation method for a hydrogen mixed gas that are capable of exerting a higher effect on health promotion.

### Solution to Problem

(1) A hydrogen mixed gas generation apparatus according to an aspect of an embodiment is characterized by comprising a warm water containment part that contains warm water that is set at a predetermined temperature, a gas supply conduit that guides a hydrogen gas that is generated by a predetermined means into the warm water in the warm water containment part, and a gas discharge conduit that guides and releases the hydrogen gas that is discharged into the warm water by the gas supply conduit from the warm water containment part to an outside thereof.
(2) Furthermore, the hydrogen mixed gas generation apparatus according to an aspect of an embodiment is characterized by further comprising a hydrogen generation part that executes one process of an electrolysis process for water, an overheat process for water vapor, or a chemical process to generate a hydrogen gas, in (1) as described above.
(3) Furthermore, the hydrogen mixed gas generation apparatus according to an aspect of an embodiment is characterized in that the warm water is maintained at 50 to 90°C, in (1) or (2) as described above.
(4) Furthermore, the hydrogen mixed gas generation apparatus according to an aspect of an embodiment is characterized by further comprising a gas discharge part that discharges a hydrogen mixed gas that includes water and is generated by passing through warm water in the warm water containment part, in any one of (1) to (3) as described above.
(5) Furthermore, a generation method for a hydrogen mixed gas according to an aspect of an embodiment is characterized by including a hydrogen gas generation step that generates a hydrogen gas by a predetermined method, and a bubbling step that passes a hydrogen gas that is generated at the hydrogen gas generation step through warm water to execute bubbling thereof.
(6) Furthermore, the generation method for a hydrogen mixed gas according to an aspect of an embodiment is characterized in that the warm water is maintained at 50 to 90°C, in (5) as described above.
(7) Furthermore, the generation method for a hydrogen mixed gas according to an aspect of an embodiment is characterized by further including a gas discharge step that discharges a hydrogen mixed gas that includes water and is generated by passing through warm water at the bubbling step, in (5) or (6) as described above. Advantageous Effects of Invention

According to an aspect of an embodiment, it is possible to generate a hydrogen mixed gas that is capable of attaining health promotion, more effectively.

### Brief Description of Drawings

FIG. 1 is an explanatory diagram that illustrates a state of use of a hydrogen mixed gas generation apparatus according to an embodiment.
FIG. 2 is a schematic and explanatory diagram of a bubbling part in a hydrogen mixed gas generation apparatus according to an embodiment.

### Description of Embodiments

Hereinafter, an embodiment(s) of a hydrogen mixed gas generation apparatus and a generation method for a hydrogen mixed gas as disclosed in the present application will be explained, with reference to the accompanying drawing(s). Additionally, this invention is not limited by an embodiment(s) as illustrated below.

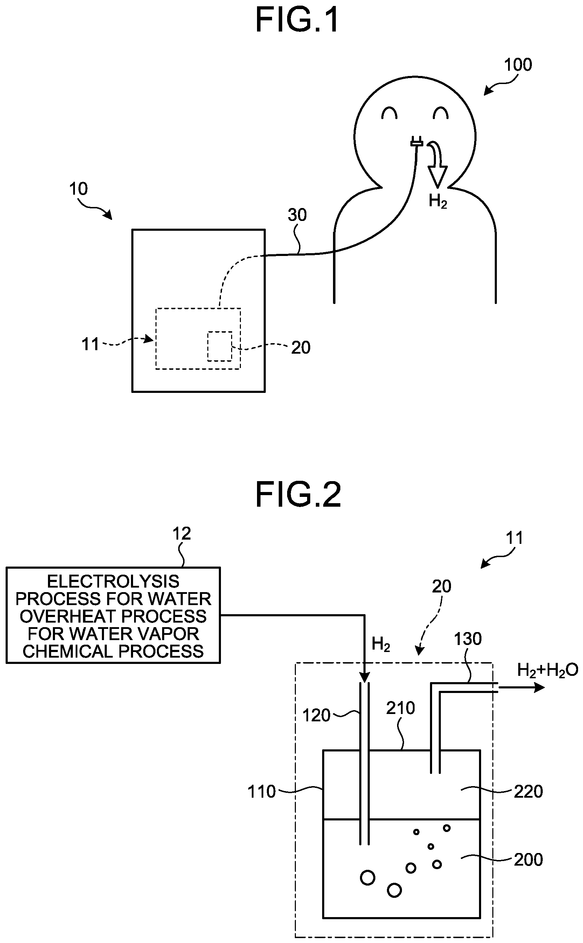

First, an outline of a configuration of a hydrogen mixed gas generation apparatus 10 for generating a hydrogen mixed gas according to the present embodiment and a generation method for a hydrogen mixed gas that is implemented by using such a hydrogen mixed gas generation apparatus 10 will be explained with reference to FIG. 1 and FIG. 2. FIG. 1 is an explanatory diagram that illustrates a state of use of the hydrogen mixed gas generation apparatus 10 according to an embodiment, and FIG. 2 is a schematic and explanatory diagram of a bubbling part in the hydrogen mixed gas generation apparatus 10 according to an embodiment.

The hydrogen mixed gas generation apparatus 10 is capable of generating a hydrogen mixed gas that includes a vapor (H₂O) and a hydrogen gas (H₂). Furthermore, the hydrogen mixed gas generation apparatus 10 according to the present embodiment is further capable of incorporating a generated hydrogen mixed gas into a human body.

As illustrated in FIG. 1, a user 100 wears a tip of a gas inhalation tube 30 that is comprised in the hydrogen mixed gas generation apparatus 10, at a nose thereof, and takes a generated hydrogen mixed gas from a nasal cavity thereof. The gas inhalation tube 30 functions as a gas discharge part as described later.

Additionally, although a user 100 that takes a hydrogen mixed gas is provided as a single person in an example as illustrated in FIG. 1, it is also possible for two or more persons to execute use by providing a plurality of available gas inhalation tubes 30 or branching the gas inhalation tube 30 into a plurality of pieces thereof. Additionally, for the gas inhalation tube(s) 30, it is possible to use, for example, a cannula(s) or the like preferably.

A hydrogen mixed gas generation unit 11 that comprises at least a bubbling part 20 is contained in an inside of a casing of the hydrogen mixed gas generation apparatus 10. As illustrated in FIG. 2, the hydrogen mixed gas generation unit 11 in the present embodiment comprises a hydrogen generation part 12 and a bubbling part 20 that passes a hydrogen gas that is generated by the hydrogen generation part 12 through warm water 200 so as to execute bubbling thereof.

The bubbling part 20 comprises a warm water containment part 110 that contains warm water 200 that is set at a predetermined temperature, a gas supply conduit 120 that guides a hydrogen gas that is generated by the hydrogen generation part 12 into the warm water 200 in the warm water containment part 110, and a gas discharge conduit 130 that guides and releases the hydrogen gas that is discharged into water by such a gas supply conduit 120 from the warm water containment part 110 to an outside thereof. For the gas supply conduit 120 and the gas discharge conduit 130, it is possible to use a tubular member that is formed of a resin such as a rubber and/or a metal.

It is sufficient that the warm water containment part 110 is, for example, a sealed and cup-shaped container where one end of the gas supply conduit 120 and one end of the gas discharge conduit 130 are inserted through a ceiling wall 210 as illustrated in FIG. 2. Then, a tip part that is one end of the gas supply conduit 120 is submerged in the warm water 200 and a starting end part that is one end of the gas discharge conduit 130 is positioned in a space part 220 between a warm water surface and the ceiling wall 210.

On the other hand, a terminal end part that is provided as another end of the gas discharge conduit 130 is connected to the gas inhalation tube 30 (FIG. 1) that is provided as a gas discharge part. Although the gas inhalation tube 30 is provided as a gas discharge part herein, the gas discharge part may be provided so as to include the gas discharge conduit 130. Furthermore, it is also possible to compose a gas discharge part by, for example, providing a fan device, a blower device, and/or the like that forcibly dispatches a hydrogen mixed gas that includes water and is generated in the bubbling part 20 to an outside thereof, and including it/them. Furthermore, it is also possible to dispatch a hydrogen gas that is generated in the hydrogen generation part 12 to an inside of the warm water containment part 110 of the bubbling part 20 forcibly by further interposing and providing a fan device, a blower device, and/or the like between the hydrogen generation part 12 and the bubbling part 20.

According to such a hydrogen mixed gas generation apparatus 10, it is possible to obtain a hydrogen mixed gas that is capable of exerting a higher effect on health promotion by undergoing a hydrogen gas generation step that generates a hydrogen gas by a predetermined method and a bubbling step that passes the hydrogen gas that is generated by such a hydrogen gas generation step through the warm water 200 so as to execute bubbling thereof.

Furthermore, the warm water 200 that is contained in the warm water containment part 110 is set at, for example, 50 to 90°C, and more preferably, is maintained at 55 to 75°C, in such a manner that it is possible for a user 100 to aspirate a hydrogen mixed gas safely and comfortably. Furthermore, if a temperature of the warm water 200 is provided in such a range, it is possible to obtain, for example, a hydrogen mixed gas that has an amount of water that is needed for vaporization thereof, even at an ordinary temperature.

Additionally, it is preferable to provide a configuration in such a manner that the warm water containment part 110 comprises a heating function and a heat-retaining function that are not illustrated in the drawing(s) in order to maintain a temperature of the warm water 200 at a desired value.

Furthermore, in the present embodiment, a hydrogen mixed gas as described above is incorporated into a human body by using the gas inhalation tube 30 that is provided as a gas discharge part. That is, a generation method for a hydrogen mixed gas in the present embodiment includes a gas discharge step that discharges a hydrogen mixed gas that includes water and is generated by passing through the warm water 200 at the bubbling step.

Hence, the hydrogen mixed gas generation apparatus 10 according to the present embodiment is configured so as to further comprise a gas discharge part that discharges a hydrogen mixed gas that includes water and is generated by passing through the warm water 200 in the warm water containment part 110, in addition to the hydrogen generation part 12 and the bubbling part 20 as has been described above.

Meanwhile, the hydrogen generation part 12 that is comprised in the hydrogen mixed gas generation unit 11 may be a device and/or a system with any configuration as long as it/they comprise(s) a function to generate hydrogen. For example, as illustrated in FIG. 2, it is possible to employ a device and/or system that is/are capable of implementing an electrolysis process for water, an overheat process for water vapor, a chemical process, or the like.

As a hydrogen mixed gas that is obtained by the hydrogen mixed gas generation apparatus 10 as has been described above is taken, it is possible to contribute to health improvement greatly. For example, as a hydrogen mixed gas that is obtained by the hydrogen mixed gas generation apparatus 10 or a generation method for a hydrogen mixed gas according to the present embodiment is taken into a subject that suffers malignancy and/or allergy or another/other disease(s), an effect on improvement in a symptom is found in many cases.

Additionally, although the hydrogen mixed gas generation apparatus 10 in an embodiment(s) as has been described above is configured so as to comprise the hydrogen generation part 12 and a gas discharge part, it is sufficient that the hydrogen mixed gas generation apparatus 10 is configured so as to comprise at least the bubbling part 20 that passes a hydrogen gas through the warm water 200 so as to execute bubbling thereof.

That is, the hydrogen generation part 12 and/or a gas discharge part may be configured so as to be separate from the hydrogen mixed gas generation apparatus 10.

A hydrogen mixed gas generation apparatus 10 and a generation method for a hydrogen mixed gas as mentioned below are realized by an embodiment(s) as has been described above.
(1) A hydrogen mixed gas generation apparatus 10 comprising a warm water containment part 110 that contains warm water 200 that is set at a predetermined temperature, a gas supply conduit 120 that guides a hydrogen gas that is generated by a predetermined means into the warm water 200 in the warm water containment part 110, and a gas discharge conduit 130 that guides and releases the hydrogen gas that is discharged into the warm water 200 by the gas supply conduit 120 from the warm water containment part 110 to an outside thereof.
   According to such a configuration, it is possible to obtain a hydrogen mixed gas that is considered to be extremely good for health maintenance of a human body efficiently.
(2) The hydrogen mixed gas generation apparatus 10 in (1) as described above, further comprising a hydrogen generation part 12 that executes one process of an electrolysis process for water, an overheat process for water vapor, or a chemical process so as to generate a hydrogen gas.
   According to such a configuration, it is also possible to execute generation of a hydrogen gas by an identical apparatus integrally.
(3) The hydrogen mixed gas generation apparatus 10 in (1) or (2) as described above, wherein the warm water 200 is maintained at 50 to 90°C.
   According to such a configuration, it is possible to obtain a hydrogen mixed gas that is readily aspirated by a user 100 and includes a vapor with a needed amount of water.
(4) The hydrogen mixed gas generation apparatus 10 in any one of (1) to (3) as described above, further comprising a gas discharge part that discharges a hydrogen mixed gas that includes water and is generated by passing through warm water 200 in the warm water containment part 110.
   According to such a configuration, it is also possible to execute a function(s) of generation of a hydrogen gas to causing a user 100 to aspirate a hydrogen mixed gas that includes a vapor by an identical apparatus integrally, so that it is possible to improve a value and/or convenience of such an apparatus.
(5) A generation method for a hydrogen mixed gas, including a hydrogen gas generation step that generates a hydrogen gas by a predetermined method, and a bubbling step that passes a hydrogen gas that is generated at the hydrogen gas generation step through warm water 200 to execute bubbling thereof.
   According to such a configuration, it is possible to obtain a hydrogen mixed gas that is considered to be extremely good for health maintenance of a human body efficiently.
(6) The generation method for a hydrogen mixed gas in (5) as described above, wherein the warm water 200 is maintained at 50 to 90°C.
   According to such a method, it is possible to obtain a hydrogen mixed gas that is readily aspirated by a user 100 and includes a vapor with a needed amount of water.
(7) The generation method for a hydrogen mixed gas in (5) or (6) as described above, further comprising a gas discharge step that discharges a hydrogen mixed gas that includes water and is generated by passing through warm water 200 at the bubbling step.

According to such a method, it is also possible to execute generation of a hydrogen gas to causing a user 100 to aspirate a hydrogen mixed gas that includes a vapor integrally.

It is possible for a person(s) skilled in the art to readily derive a variation(s) and/or an additional effect(s) in an embodiment(s) as described above. Hence, a broader aspect(s) of the present invention is/are not limited to a specific detail(s) and a representative embodiment(s) as illustrated and described above. Therefore, various modifications are possible without departing from the spirit or scope of a general inventive concept that is defined by the appended claim(s) and an equivalent(s) thereof.

### Reference Signs List

- 10: hydrogen mixed gas generation apparatus
- 11: hydrogen mixed gas generation unit
- 12: hydrogen generation part
- 20: bubbling part
- 30: gas inhalation tube
- 110: warm water containment part
- 120: gas supply conduit
- 130: gas discharge conduit
- 200: warm water
- 210: ceiling wall
- 220: space part

## Claims

1. A hydrogen mixed gas generation apparatus, **characterized by** comprising:
a warm water containment part that contains warm water that is set at a predetermined temperature;
a gas supply conduit that guides a hydrogen gas that is generated by a predetermined means into the warm water in the warm water containment part; and
a gas discharge conduit that guides and releases the hydrogen gas that is discharged into the warm water by the gas supply conduit from the warm water containment part to an outside thereof.

2. The hydrogen mixed gas generation apparatus according to claim 1, **characterized by** further comprising
a hydrogen generation part that executes one process of an electrolysis process for water, an overheat process for water vapor, or a chemical process to generate a hydrogen gas.

3. The hydrogen mixed gas generation apparatus according to claim 1 or 2, **characterized in that**
the warm water is maintained at 50 to 90°C.

4. The hydrogen mixed gas generation apparatus according to any one of claims 1 to 3, **characterized by** further comprising
a gas discharge part that discharges a hydrogen mixed gas that includes water and is generated by passing through warm water in the warm water containment part.

5. A generation method for a hydrogen mixed gas, **characterized by** including:
a hydrogen gas generation step that generates a hydrogen gas by a predetermined method; and
a bubbling step that passes a hydrogen gas that is generated at the hydrogen gas generation step through warm water to execute bubbling thereof.

6. The generation method for a hydrogen mixed gas according to claim 5, **characterized in that**
the warm water is maintained at 50 to 90°C.

7. The generation method for a hydrogen mixed gas according to claim 5 or 6, **characterized by** further including
a gas discharge step that discharges a hydrogen mixed gas that includes water and is generated by passing through warm water at the bubbling step.
